(19) Europäisches Patentamt
European Patent Office
Office européen des brevets

(11) EP 3 639 863 B1

(12) **EUROPEAN PATENT SPECIFICATION**

(45) Date of publication and mention of the grant of the patent:
**20.07.2022 Bulletin 2022/29**

(21) Application number: **19203007.0**

(22) Date of filing: **14.10.2019**

(51) International Patent Classification (IPC):
***A61L 29/08*** *(2006.01)* ***C09D 4/00*** *(2006.01)*

(52) Cooperative Patent Classification (CPC):
(C-Sets available)
**C09D 4/00; A61L 29/085; A61L 29/14;**
A61L 2400/10; C08G 77/24; C08G 77/46 (Cont.)

(54) **SURFACE MODIFICATION METHOD AND GASKET FOR SYRINGE**

OBERFLÄCHENMODIFIZIERUNGSVERFAHREN UND DICHTUNG FÜR SPRITZE

PROCÉDÉ DE MODIFICATION DE SURFACE ET JOINT D'ÉTANCHÉITÉ POUR SERINGUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2018 JP 2018194297**

(43) Date of publication of application:
**22.04.2020 Bulletin 2020/17**

(73) Proprietor: **Sumitomo Rubber Industries, Ltd.**
**Kobe-shi,**
**Hyogo-ken 651-0072 (JP)**

(72) Inventor: **MINAGAWA, Yasuhisa**
**Kobe-shi, Hyogo-ken, 651-0072 (JP)**

(74) Representative: **Manitz Finsterwald**
**Patent- und Rechtsanwaltspartnerschaft mbB**
**Martin-Greif-Strasse 1**
**80336 München (DE)**

(56) References cited:
**EP-A1- 3 173 106**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 29/085, C08L 83/00;**
**C09D 183/08, C08L 83/00;**
**C09D 183/08, C08L 83/00, C08L 83/00**

## Description

### TECHNICAL FIELD

The present invention relates to a surface modification method and a gasket for syringes at least part of whose surface is modified by the modification method.

### BACKGROUND ART

In view of the importance of sealing properties (liquid leakage resistance), elastic bodies such as rubber are used in parts which slide while maintaining a seal, e.g., a gasket which is integrated with a plunger of a syringe to form a seal between the plunger and the barrel. Unfortunately, such elastic bodies have a slight problem with sliding properties (see Patent Literature 1). To address this problem, a sliding property-improving agent, for example silicone oil, may be applied to the sliding surface; however, a concern has been raised over the potential adverse effects of silicone oil on recently marketed bio-preparations. On the other hand, gaskets not coated with a sliding property-improving agent have inferior sliding properties and therefore do not allow plungers to be smoothly pushed but cause them to pulsate during administration. This can result in problems such as inaccurate injection amounts and infliction of pain on patients.

To satisfy the conflicting requirements, i.e., sealing properties and sliding properties, a method of coating surfaces with a self-lubricating PTFE film has been proposed (see Patent Literature 2). Unfortunately, such PTFE films are generally expensive and thus will increase the production cost of processed products, limiting the range of application of the method. Moreover, products coated with PTFE films might be unreliable when they are used in applications where sliding or similar movement is repeated and durability is therefore required. Still another problem is that since PTFE is vulnerable to radiation, PTFE-coated products cannot be sterilized by radiation.

Consideration may also be given to the use in other applications where sliding properties are required in the presence of water. Specifically, water can be delivered without a loss by reducing the fluid resistance of the inner surface of a pre-filled syringe or of the inner surface of a pipe or tube for delivering water, or by increasing or markedly reducing the contact angle with water thereof. Reducing the surface resistance of the internal/external surface of a catheter tube may facilitate insertion of the catheter into the body or introduction of a guide wire through the catheter. Drainage of water on wet roads or of snow on snowy roads can be improved by reducing the fluid resistance of the groove surfaces of tires, or by increasing or markedly reducing the contact angle with water thereof. This can result in improved grip performance and hydroplaning performance and thus better safety. In addition, less adhesion of dirt and dust can be expected when the sliding resistance of the sidewall surfaces of tires or the walls of buildings is reduced, or when the contact angle with water thereof is increased.

Further advantageous effects can be expected, including, for example: less pressure loss upon delivering liquid such as water or an aqueous solution through a diaphragm such as a diaphragm pump or valve; easy sliding of skis and snowboards achieved by enhancing the sliding properties of the sliding surfaces thereof; better noticeability of road signs and signboards achieved by enhancing the sliding properties thereof to allow snow to readily slide on the surface; reduction in water resistance or drag on the outer peripheries of ships and less adhesion of bacteria to these outer peripheries achieved by reducing the sliding resistance of the outer peripheries or by increasing the contact angle with water thereof; and reduction in water resistance or drag of swimsuits achieved by improving the sliding properties of the thread surfaces thereof.

Patent Literature 3 describes that a natural rubber catheter is first immersed in a butoxy/ethoxy tetraalkoxysilane, taken out, dried, then immersed in a solution of a silane compound containing a fluorine-containing hydrophobic group, taken out and allowed to stand in order to obtain a surface-treated catheter. Document EP 3 173 106 discloses a method in which a rubber surface is first treated with a primer consisting in a silane and then with a fluoroalkyl group containing silane.

### CITATION LIST

### PATENT LITERATURE

Patent Literature 1: JP 2004-298220 A
Patent Literature 2: JP 2010-142573 A
Patent Literature 3: EP 3 173 106 A1

## SUMMARY OF INVENTION

### TECHNICAL PROBLEM

The present invention aims to solve the above problems and provide a method for modifying a surface of a rubber vulcanizate and a gasket for syringes, which can provide a variety of functions, including sliding properties, liquid leakage resistance, and protein adsorption resistance.

### SOLUTION TO PROBLEM

The present invention relates to a surface modification method for modifying a surface of a rubber vulcanizate having a Shore A hardness of 55 to 90 as a modification target, the Shore A hardness being determined using a type-A durometer at 23°C in accordance with JIS K 6253 "Rubber, vulcanized or thermoplastic - Determination of hardness", the method including: step 1 of adding a silane compound to the surface of the modification target; and step 2 of reacting at least a fluoroalkyl group-containing silane compound and a perfluoroether group-containing silane compound to form a surface-modified layer.

Preferably, the fluoroalkyl group-containing silane compound and the perfluoroether group-containing silane compound are combined in a ratio of 1:99 to 100:0.

Preferably, the fluoroalkyl group is represented by the following formula:

$$F_3C\text{-}(CF_2)_n\text{-}(CH_2)_m\text{-}$$

wherein n is 0 to 5, and m is 0 to 8.

Preferably, the fluoroalkyl group-containing silane compound is represented by the following formula (1):

$$F_3C\text{-}(CF_2)_n\text{-}(CH_2)_m\text{-}Si(OR^1)_3 \qquad (1)$$

wherein n is 0 to 5; m is 0 to 8; and each $R^1$ may be the same or different and represents an alkyl group.

Preferably, the perfluoroether group-containing silane compound is represented by the following formula (2) or (3):

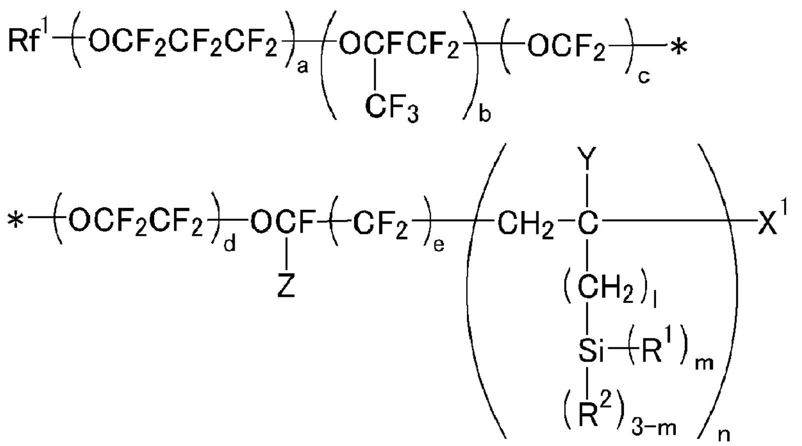

(2)

wherein $Rf^1$ is a perfluoroalkyl group; Z is fluorine or a trifluoromethyl group; a, b, c, d, and e are the same as or different from each other and each represent an integer of 0 or 1 or more, provided that a + b + c + d + e is 1 or more and the order of the repeating units parenthesized by subscripts a, b, c, d, and e occurring in the formula is not limited to that shown; Y is hydrogen or a C1-C4 alkyl group; $X^1$ is hydrogen, bromine, or iodine; $R^1$ is a hydroxy group or a hydrolyzable substituent; $R^2$ is hydrogen or a monovalent hydrocarbon group; 1 is 0, 1, or 2; m is 1, 2, or 3; n is an integer of 1 or more; and the two ends marked by * are directly bonded to each other, or

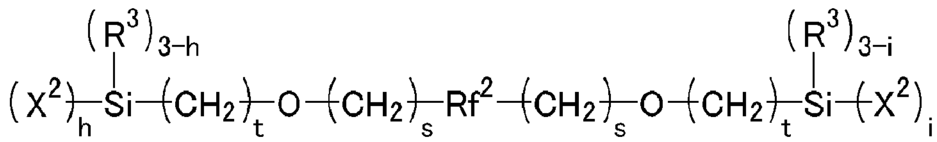 (3)

wherein $Rf^2$ is a divalent group that contains a unit represented by $-(C_kF_{2k})O-$ where k is an integer of 1 to 6, and has a non-branched linear perfluoropolyalkylene ether structure; each $R^3$ is the same or different and represents a C1-C8 monovalent hydrocarbon group; each $X^2$ is the same or different and represents a hydrolyzable group or a halogen atom; each s is the same or different and represents an integer of 0 to 2; each t is the same or different and represents an integer of 1 to 5; and h and i are the same as or different from each other and each represent 1, 2, or 3.

Preferably, the surface-modified layer formed by the surface modification method has a thickness of 30 to 500 nm.

Another aspect of the present invention relates to a gasket for syringes, at least partly including the surface-modified layer formed by the surface modification method.

Preferably, the gasket for syringes has a sliding surface provided with a plurality of annular projections, the annular projections include a first projection nearest to a top surface of the gasket, and the first projection has a surface roughness Ra of not greater than 1.0, wherein the surface roughness Ra is the arithmetic average height Ra defined in JIS B 0601-2001 or ISO 4287-1997.

Preferably, the surface roughness Ra is not greater than 0.8.

Preferably, the surface roughness Ra is not greater than 0.6.

ADVANTAGEOUS EFFECTS OF INVENTION

The surface modification method of the present invention which includes steps 1 and 2 can cost-effectively impart a variety of functions, including sliding properties, liquid leakage resistance, and protein adsorption resistance.

BRIEF DESCRIPTION OF DRAWINGS

Fig. 1 illustrates an example of a longitudinal sectional view of a gasket base material on which a surface-modified layer is to be formed.

Fig. 2 illustrates an example of a longitudinal sectional view of a gasket for syringes in which a surface-modified layer is formed on the surface of the gasket base material.

DESCRIPTION OF EMBODIMENTS

The present invention relates to a surface modification method for modifying a surface of a rubber vulcanizate having a Shore A hardness of 55 to 90 as a modification target, the Shore A hardness being determined using a type-A durometer at 23°C in accordance with JIS K 6253 "Rubber, vulcanized or thermoplastic - Determination of hardness", wherein the method includes: step 1 of adding a silane compound to the surface of the modification target; and step 2 of reacting at least a fluoroalkyl group-containing silane compound and a perfluoroether group-containing silane compound to form a surface-modified layer.

When one desires to impart functions to a rubber vulcanizate generally having a very rough surface by forming polymer chains on the surface of the rubber vulcanizate, the functions can be significantly achieved by providing polymer chains on the surface of the rubber vulcanizate whose surface roughness is reduced by increasing its hardness and reducing its shrinkage after vulcanization. The hardness of the rubber may be increased, for example, by incorporating a large amount of filler or increasing the crosslink density. Moreover, functions such as sliding properties can be more easily achieved (or more improved) when polymer chains are provided on the rubber having an increased hardness.

In this context, the present invention provides a surface modification method which first increases the hardness of the surface of a modification target, adds a silane compound to the resulting surface, and further reacts (e.g., adds) at least a fluoroalkyl group-containing silane compound with the silane compound to treat the surface, whereby a functional fluoroalkyl group-containing silane compound is provided on the outermost surface. It should be noted that desired properties such as sufficient sliding properties cannot be achieved when a rubber surface having a low hardness is treated similarly, i.e., by only providing a functional fluoroalkyl group-containing silane compound.

Moreover, when the surface of a modification target is modified so that a fluoroalkyl group-containing silane compound with low surface free energy is provided on the outermost surface, high properties such as sliding properties,

liquid leakage resistance, biocompatibility, and protein adsorption resistance can be imparted to the modification target. It should be noted that perfluoroether groups also have low surface free energy but contain ether oxygen, whereas in the present invention an oxygen-free fluoroalkyl group is dominantly provided on the surface, which can provide higher properties such as liquid leakage resistance, biocompatibility, and protein adsorption resistance.

Step 1 includes adding a silane compound to a surface of a modification target (rubber vulcanizate).

Examples of rubbers (vulcanized rubbers) that can be used as the modification target include diene rubbers such as styrene-butadiene rubber, polybutadiene rubber, polyisoprene rubber, natural rubber, and deproteinized natural rubber; and butyl rubber and halogenated butyl rubber which have a degree of unsaturation of a few percent of isoprene units. The butyl rubber or halogenated butyl rubber, if used, is preferably a rubber crosslinked by triazine because the amount of matter extracted from the rubber vulcanizate is reduced. In this case, the rubber may contain an acid acceptor. Examples of suitable acid acceptors include hydrotalcite and magnesium carbonate.

If other rubbers are used, preferably sulfur vulcanization is performed. In such cases, compounding ingredients commonly used in sulfur vulcanization may be added, such as vulcanization accelerators, zinc oxide, fillers, and silane coupling agents. Examples of suitable fillers include carbon black, silica, clay, talc, and calcium carbonate.

The vulcanization conditions of the rubber used may be selected appropriately. The rubber is preferably vulcanized at a temperature of 150°C or higher, more preferably 170°C or higher, still more preferably 175°C or higher.

In view of functions such as sliding properties, liquid leakage resistance, and protein adsorption resistance, the rubber vulcanizate (modification target) has a Shore A hardness of 55 to 90, preferably 60 to 85.

The hardness of the rubber vulcanizate is determined using a type-A durometer (Shore A) at 23°C in accordance with JIS K 6253.

Non-limiting examples of the silane compound include silane compounds containing no fluoroalkyl group. In particular, to better achieve the advantageous effects, alkoxysilanes and modified alkoxysilanes are preferred, with alkoxysilanes being more preferred. These silane compounds may be used alone or in combinations of two or more.

Examples of the alkoxysilanes include monoalkoxysilanes such as trimethylmethoxysilane, triethylethoxysilane, tripropylpropoxysilane, and tributylbutoxysilane; dialkoxysilanes such as dimethyldimethoxysilane, diethyldiethoxysilane, dipropyldipropoxysilane, and dibutyldibutoxysilane; trialkoxysilanes such as methyltrimethoxysilane, ethyltriethoxysilane, propyltripropoxysilane, and butyltributoxysilane; and tetraalkoxysilanes such as tetramethoxysilane, tetraethoxysilane, tetrapropoxysilane, tetrabutoxysilane, dibutoxydiethoxysilane, butoxytriethoxysilane, and ethoxytributoxysilane. These may be used alone or in combinations of two or more. To better achieve the advantageous effects, tetraalkoxysilanes are preferred among these, with tetramethoxysilane, tetraethoxysilane, tetrabutoxysilane, dibutoxydiethoxysilane, butoxytriethoxysilane, and ethoxytributoxysilane being more preferred.

The term "modified alkoxysilane" refers to an alkoxysilane having a substituent such as an amino, carboxyl, hydroxy, or epoxy group, and preferably contains at least one selected from the group consisting of alkyl, amino, carboxyl, hydroxy, and epoxy groups.

To better achieve the advantageous effects, alkoxysilanes and modified alkoxysilanes each having a carbon number of 4 to 22, preferably 4 to 16, are preferred.

To better achieve the advantageous effects, alkoxysilanes and modified alkoxysilanes each containing at least one selected from the group consisting of methoxy, ethoxy, propoxy, and butoxy groups, preferably ethoxy and/or butoxy groups, still more preferably ethoxy and butoxy groups, are preferred.

Examples of usable commercial products of the silane compound include Primer coat PC-3B (Fluoro Technology, the butoxy/ethoxy tetraalkoxysilane represented by the following formula:

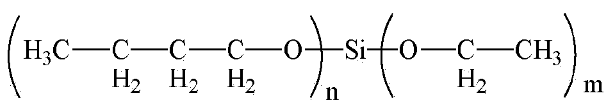

wherein m + n = 4 with n > m > 0 on average).

In step 1, the silane compound may be added to the surface of the rubber vulcanizate (modification target) by any method. Appropriate conventional methods may be employed, such as bringing the modification target into contact with the silane compound.

In step 2, the product (silane compound-treated product) obtained by adding the silane compound to the surface of the modification target in step 1 is further reacted with a fluoroalkyl group-containing silane compound to form a surface-modified layer. Thus, the fluoroalkyl group-containing silane compound is added to (reacted with) the silane compound provided on the surface of the modification target to form a surface-modified layer (modified surface) on the surface of the modification target.

The fluoroalkyl group of the fluoroalkyl group-containing silane compound may be, for example, a group represented by the following formula:

$F_3C-(CF_2)_n-(CH_2)-$

wherein n is 0 to 5, and m is 0 to 8.

In the formula, n is preferably 1 to 5, more preferably 3 to 5; m is preferably 1 to 6, more preferably 2 to 6; and m and n preferably satisfy $0 \leq m + n \leq 10$, more preferably $0 \leq m + n \leq 7$.

Specific examples of the fluoroalkyl group include 3,3,3-trifluoropropyl, 3,3,4,4,4-pentafluorobutyl, 3,3,4,4,5,5,6,6,6-nonafluorohexyl, 3,3,4,4,5,5,6,6,7,7,7-undecafluoroheptyl, and 3,3,4,4,5,5,6,6,7,7,8,8,8-tridecafluorooctyl groups.

To cost-effectively impart a variety of functions, including sliding properties, liquid leakage resistance, and protein adsorption resistance, the fluoroalkyl group-containing silane compound may suitably be, but not limited to, a compound represented by the following formula (1):

$$F_3C-(CF_2)_n-(CH_2)_m-Si(OR^1)_3 \qquad (1)$$

wherein n is 0 to 5; m is 0 to 8; and each $R^1$ may be the same or different and represents an alkyl group.

In formula (1), n is preferably 1 to 5, more preferably 3 to 5; m is preferably 1 to 6, more preferably 2 to 6; and m and n preferably satisfy $0 \leq m + n \leq 10$, more preferably $0 \leq m + n \leq 7$. $R^1$ (alkyl group) may be linear, branched, or cyclic, or a combination of two or more of these structures. The number of carbon atoms of $R^1$ is preferably 1 to 10, more preferably 1 to 5, still more preferably 1 to 3. Examples of the alkyl group for $R^1$ include methyl, ethyl, and propyl groups.

Specific examples of the fluoroalkyl group-containing silane compound of formula (1) include 3,3,3-trifluoropropyltrimethoxysilane, 3,3,3-trifluoropropyltriethoxy-silane, 3,3,4,4,5,5,6,6,7,7,8,8,8-tridecafluoro-octyltrimethoxysilane, triethoxy-1H,1H,2H,2H-tridecafluoro-n-octylsilane, and $CF_3(CF_2)_3CH_2CH_2Si(OCH_2CH_3)_3$. These may be used alone or in combinations of two or more.

In step 2, the fluoroalkyl group-containing silane compound may be reacted with (added to) the silane compound-treated product by any method. Appropriate conventional methods may be employed, such as bringing a solution of the fluoroalkyl group-containing silane compound into contact with the silane compound-treated product. The solution of the fluoroalkyl group-containing silane compound may be prepared by appropriately adjusting the concentration of the compound in a known solvent that can dissolve the compound, such as water, perfluorohexane, acidic water, methanol, ethanol, or a mixture of water with methanol or ethanol. The contact between the solution and the silane compound-treated product may be made by any method that brings them into contact with each other, such as application, spraying, or immersion.

The reaction of the fluoroalkyl group-containing silane compound with the silane compound-treated product is preferably further held at a humidity of 50% or higher after the contact, e.g., immersion. This further promotes the reaction so that the advantageous effects can be well achieved. The humidity is more preferably 60% or higher, still more preferably 80% or higher. The upper limit of the humidity is not particularly limited, but is preferably, for example, 100% or lower. The holding time and temperature may be appropriately chosen and are preferably, for example, 0.5 to 60 hours and 20 to 120°C, respectively.

To achieve the advantageous effects well, step 2 includes reacting (adding) not only the fluoroalkyl group-containing silane compound but also a perfluoroether group-containing silane compound with the silane compound-treated product obtained in step 1 to form a surface-modified layer.

The perfluoroether group-containing silane compound may be any silane compound having a perfluoroether group. Suitable examples include compounds represented by the following formula (2) or (3):

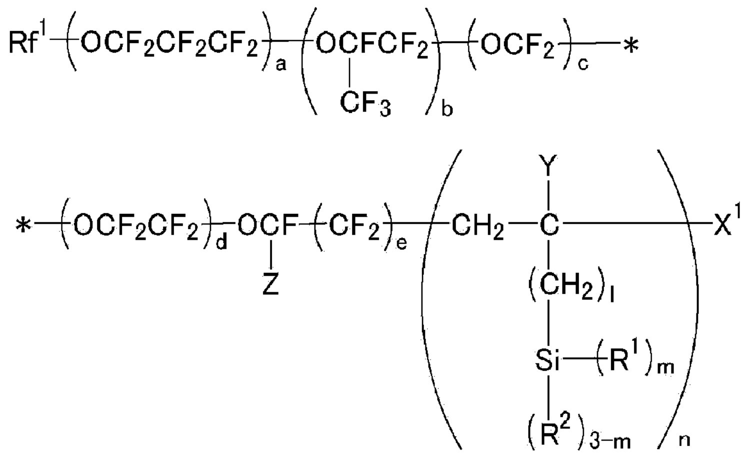

(2)

wherein $Rf^1$ is a perfluoroalkyl group; Z is fluorine or a trifluoromethyl group; a, b, c, d, and e are the same as or different from each other and each represent an integer of 0 or 1 or more, provided that a + b + c + d + e is 1 or more and the order of the repeating units parenthesized by subscripts a, b, c, d, and e occurring in the formula is not limited to that shown; Y is hydrogen or a C1-C4 alkyl group; $X^1$ is hydrogen, bromine, or iodine; $R^1$ is a hydroxy group or a hydrolyzable substituent such as a C1-C4 alkoxy group; $R^2$ is hydrogen or a monovalent hydrocarbon group; 1 is 0, 1, or 2; m is 1, 2, or 3; n is an integer of 1 or more; and the two ends marked by * are directly bonded to each other, or

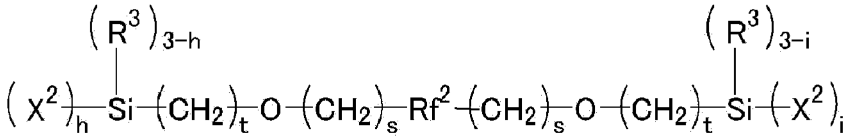

(3)

wherein $Rf^2$ is a divalent group that contains a unit represented by $-(C_kF_{2k})O-$ where k is an integer of 1 to 6, and has a non-branched linear perfluoropolyalkylene ether structure; each $R^3$ is the same or different and represents a C1-C8 monovalent hydrocarbon group; each $X^2$ is the same or different and represents a hydrolyzable group such as a C1-C4 alkoxy group or a halogen atom; each s is the same or different and represents an integer of 0 to 2; each t is the same or different and represents an integer of 1 to 5; and h and i are the same as or different from each other and each represent 1, 2, or 3.

$Rf^1$ in formula (2) may be any perfluoroalkyl group that can be used to form a common organic-containing fluoropolymer, and examples include linear or branched C1-C16 groups. In particular, $CF_3$-, $C_2F_5$-, and $C_3F_7$- are preferred.

In formula (2), each of a, b, c, d, and e represents the number of repeating units in the perfluoropolyether chain which constitutes the backbone of the fluorine-containing silane compound, and is independently preferably 0 to 200, more preferably 0 to 50. Moreover, a + b + c + d + e (the sum of a to e) is preferably 1 to 100. The order of the repeating units parenthesized by subscripts a, b, c, d, and e occurring in formula (2) is not limited to the order shown, and the repeating units may be joined in any order.

Examples of the C1-C4 alkyl group for Y in formula (2) include methyl, ethyl, propyl, and butyl groups, which may be linear or branched. When $X^1$ is bromine or iodine, the fluorine-containing silane compound can easily form a chemical bond.

Preferred examples of the hydrolyzable substituent for $R^1$ in formula (2) include, but not limited to, halogens, $-OR^4$, $-OCOR^4$, $OC(R^4)=C(R^5)_2$, $-ON=C(R^4)_2$, and $-ON=CR^6$, where $R^4$ is an aliphatic hydrocarbon group or an aromatic hydrocarbon group; $R^5$ is hydrogen or a C1-C4 aliphatic hydrocarbon group; and $R^6$ is a C3-C6 divalent aliphatic hydrocarbon group. The hydrolyzable substituent is more preferably chlorine, $-OCH_3$, or $-OC_2H_5$. Preferred examples of the monovalent hydrocarbon group for $R^2$ include, but not limited to, methyl, ethyl, propyl, and butyl groups, which may be linear or branched.

In formula (2), 1 represents the number of carbon atoms of the alkylene group between the carbon in the perfluoropolyether chain and the silicon attached thereto and is preferably 0; and m represents the number of substituents $R^1$ bonded to the silicon to which $R^2$ is bonded through a bond not attached to $R^1$. The upper limit of n is not particularly limited, but is preferably an integer of 1 to 10.

In formula (3), the group $Rf^2$ is preferably, but not limited to, such that when each s is 0, the ends of $Rf^2$ group bonded to the oxygen atoms in formula (3) are not oxygen atoms. Moreover, k in $Rf^2$ is preferably an integer of 1 to 4.

Specific examples of the group $Rf^2$ include - $CF_2CF_2O(CF_2CF_2CF_2O)_jCF_2CF_2$- where j is an integer of 1 or more, preferably of 1 to 50, more preferably of 10 to 40; and -$CF_2(OC_2F_4)_p$-$(OCF_2)_q$- where p and q are each an integer of 1 or more, preferably of 1 to 50, more preferably of 10 to 40, and the sum of p and q is an integer of 10 to 100, preferably of 20 to 90, more preferably of 40 to 80, and the repeating units $(OC_2F_4)$ and $(OCF_2)$ are randomly arranged.

$R^3$ in formula (3) is preferably a C1-C8 monovalent hydrocarbon group, and examples include alkyl groups such as methyl, ethyl, propyl, butyl, pentyl, hexyl, heptyl, and octyl groups; cycloalkyl groups such as cyclopentyl and cyclohexyl groups; aryl groups such as phenyl, tolyl, and xylyl groups; aralkyl groups such as benzyl and phenethyl groups; and alkenyl groups such as vinyl, allyl, butenyl, pentenyl, and hexenyl groups. Preferred among these is a methyl group.

Examples of the hydrolyzable group for $X^2$ in formula (3) include alkoxy groups such as methoxy, ethoxy, propoxy, and butoxy groups; alkoxyalkoxy groups such as methoxymethoxy, methoxyethoxy, and ethoxyethoxy groups; alkenyloxy groups such as allyloxy and isopropenoxy groups; acyloxy groups such as acetoxy, propionyloxy, butylcarbonyloxy, and benzoyloxy groups; ketoxime groups such as dimethylketoxime, methylethylketoxime, diethylketoxime, cyclopennoxime, and cyclohexanoxime groups; amino groups such as N-methylamino, N-ethylamino, N-propylamino, N-butylamino, N,N-dimethylamino, N,N-diethylamino, and N-cyclohexylamino groups; amide groups such as N-methylacetamide, N-ethylacetamide, and N-methylbenzamide groups; and aminooxy groups such as N,N-dimethylaminooxy and N,N-diethylaminooxy groups. Examples of the halogen atom for $X^2$ include chlorine, bromine, and iodine atoms. Preferred among these are a methoxy group, an ethoxy group, an isopropenoxy group, and a chlorine atom.

In formula (3), s is preferably 1, and t is preferably 3. In view of hydrolyzability, h and i are each preferably 3.

The perfluoroether group-containing silane compound may also be a compound represented by the following formula (4) :

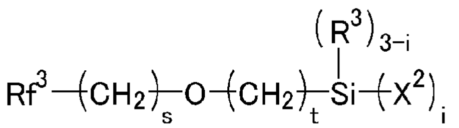

(4)

wherein Rf is a monovalent group that contains a unit represented by -$(C_kF_{2k})$O- where k is an integer of 1 to 6, and has a non-branched linear perfluoropolyalkylene ether structure; each $R^3$ is the same or different and represents a C1-C8 monovalent hydrocarbon group; each $X^2$ is the same or different and represents a hydrolyzable group such as a C1-C4 alkoxy group or a halogen atom; s represents an integer of 0 to 2; t represents an integer of 1 to 5; and i represents 1, 2, or 3.

In formula (4), the group $Rf^3$ is preferably, but not limited to, such that when s is 0, the end of $Rf^3$ group bonded to the oxygen atom in formula (4) is not an oxygen atom. Moreover, k in $Rf^3$ is preferably an integer of 1 to 4. Specific examples of the group $Rf^3$ include $CF_3CF_2O(CF_2CF_2CF_2O)_jCF_2CF_2$- where j is an integer of 1 or more, preferably of 1 to 50, more preferably of 10 to 40; and $CF_3(OC_2F_4)_p$-$(OCF_2)_q$- where p and q are each an integer of 1 or more, preferably of 1 to 50, more preferably of 10 to 40, and the sum of p and q is an integer of 10 to 100, preferably of 20 to 90, more preferably of 40 to 80, and the repeating units $(OC_2F_4)$ and $(OCF_2)$ are randomly arranged.

Examples of $R^3$ in formula (4) include those mentioned for $R^3$ in formula (3). Examples of $X^2$ in formula (4) include those mentioned for $X^2$ in formula (3). In formula (4), s is preferably 1, and t is preferably 3. In view of hydrolyzability, i in formula (4) is preferably 3.

For durable mold-releasing effect, the perfluoroether group-containing silane compound preferably has an average molecular weight of 1,000 to 10,000. The average molecular weight may be determined by gel permeation chromatography (GPC) calibrated with polystyrene standards.

Examples of commercial products of the perfluoroether group-containing silane compound include OPTOOL DSX (Daikin Industries, Ltd.), KY-108 and KY-164 (Shin-Etsu Chemical Co., Ltd.), Fluorolink S10 (Solvay Specialty Polymers Japan K.K.), Novec 2702 and Novec 1720 (3M Japan Limited), and FLUOROSURF series such as FLUOROSURF FG-5080SH (Fluoro Technology), and SIP6720.72 (Gelest, [perfluoro(polypropyleneoxy)]methoxypropyltrimethoxysilane, $CF_3CF_2CF_2O(CF_2CF_2CF_2O)_nCH_2OCH_2CH_2CH_2Si(OCH_3)_3$).

In step 2, the perfluoroether group-containing silane compound may further be reacted (added) by any method. For example, the reaction may be carried out as described for the fluoroalkyl group-containing silane compound, except that a solution containing not only the fluoroalkyl group-containing silane compound but also the perfluoroether group-containing silane compound is used instead of the solution of the fluoroalkyl group-containing silane compound.

In step 2, to impart a variety of functions, including sliding properties, liquid leakage resistance, and protein adsorption resistance, the fluoroalkyl group-containing silane compound and the perfluoroether group-containing silane compound are preferably combined in a ratio (mass ratio of fluoroalkyl group-containing silane compound:perfluoroether group-containing silane compound) of 1:99 to 100:0, more preferably 30:70 to 100:0, still more preferably 60:40 to 100:0.

The thickness of the resulting surface-modified layer (the thickness of the entire surface-modified layer formed by addition (reaction) of the silane compound, fluoroalkyl group-containing silane compound, and optional perfluoroether

group-containing silane compound) is preferably 30 to 500 nm, more preferably 50 to 350 nm. When the thickness is less than 30 nm, good sliding properties tend not to be achieved. When the thickness is more than 500 nm, a further improvement in sliding properties tends not to be expected, and sealing properties also tend to decrease.

In step 1, two or more silane compounds may be simultaneously added. In step 2, two or more fluoroalkyl group-containing silane compounds or perfluoroether group-containing silane compounds may be simultaneously reacted with the surface of the silane compound-treated product obtained in step 1. Moreover, each combination of silane compounds may be stacked in two or more layers.

According to the surface modification method, a functional fluoroalkyl group-containing silane compound is provided on the outermost surface by adding a silane compound to a surface of a modification target (rubber vulcanizate) having a predetermined hardness and then reacting (e.g., adding) at least a fluoroalkyl group-containing silane compound with the surface to which the silane compound is added, to form a surface-modified layer. Thus, desired functions can be imparted very cost-effectively. Further, the attachment of at least a functional fluoroalkyl group-containing silane compound to the outermost surface provides desired properties such as high sliding properties, liquid leakage resistance, biocompatibility, and protein adsorption resistance.

The surface modification method may be applied to a rubber vulcanizate to produce a surface-modified elastic body. For example, surface-modified elastic bodies that are excellent in sliding properties in the presence of water or in a dry state can be obtained. Such surface-modified elastic bodies are also excellent in that they have low friction and low water resistance or drag. Moreover, the method may be applied to at least a part of a three-dimensional solid body (e.g., elastic body) to produce a surface-modified elastic body on which a surface-modified layer is formed.

The surface modification method may also be applied to a rubber vulcanizate to produce a gasket for syringes at least partly having a surface-modified layer (a gasket for syringes in which a surface-modified layer is formed on at least a part of the surface of a modification target). The surface-modified layer is preferably formed at least on the sliding portion of the surface of the gasket, or may be formed on the entire surface thereof.

Suitable examples of the gasket for syringes include a gasket for syringes having a sliding surface provided with a plurality of annular projections, wherein the annular projections include a first projection nearest to the top surface of the gasket, and the first projection has a surface roughness Ra of not greater than 1.0. When the surface-modified layer is formed on the surface of a gasket base material, and the surface roughness Ra of at least the first projection nearest to the top surface of the resulting gasket is adjusted to not greater than 1.0, high sliding properties and high liquid leakage resistance can be simultaneously achieved.

Fig. 1 illustrates an example of a longitudinal sectional view (cross-sectional view in the sliding direction, longitudinal profile) of a base material 1 (gasket base material 1) on which a surface-modified layer is to be formed. Fig. 2 illustrates an example of a longitudinal sectional view of a gasket for syringes 2 in which a surface-modified layer 21 is formed on the surface of the gasket base material 1 of Fig. 1.

The gasket for syringes 2 may be used in, for example, a syringe that includes a barrel into which liquid is to be injected, a plunger for pushing the injected liquid out of the barrel, and a gasket attached to the tip of the plunger.

The gasket for syringes 2 of Fig. 2 is one in which a surface-modified layer 21 is formed on at least a part of the sliding surface of the gasket base material 1 of Fig. 1. In the cylindrical gasket base material 1 and the gasket for syringes 2 in which the surface-modified layer 21 is formed on the gasket base material 1, the circumferences of the top surface 12 on the liquid-contact side and of the bottom surface 13 to be connected to the tip of a plunger are integrated with a sliding portion 14 (cylindrical portion) extending in the height direction (sliding direction).

With regard to the gasket base material 1 and the gasket for syringes 2, the outer periphery of the sliding portion 14 includes three annular projections that make a sliding contact with the inner periphery of the cylindrical portion of the barrel; specifically, a first projection 14a at a position nearest to the top surface 12 (first projection 14a nearest to the top surface), a bottom-side projection 14c at a position farthest from the top surface 12 (bottom-side projection 14c nearest to the bottom surface), and an intermediate projection 14b between the projections 14a and 14c. In the gasket base material of Fig. 1, the top surface 12 is integrated with the first projection 14a.

Although Figs. 1 and 2 show embodiments having three annular projections, there may be any number, but at least two, of annular projections. Although the embodiments have one intermediate projection 14b, any projection between the first projection and the bottom-side projection corresponds to an intermediate projection, and there may be a plurality of intermediate projections.

To simultaneously achieve sliding properties and liquid leakage resistance, the gasket for syringes 2 preferably has three or more annular projections. In the cylindrical gasket base material 1 and the gasket for syringes 2, the top surface 12 on the liquid-contact side, the bottom surface 13 to be connected to the tip of a plunger, the first projection 14a, the intermediate projection 14b, the bottom-side projection 14c, and the sliding portion 14 may each have any shape.

The gasket for syringes 2 of Fig. 2 is one in which a surface-modified layer 21 is formed on at least a part of the surface of the gasket base material 1. The figure illustrates an example in which the surface-modified layer 21 is formed on the top surface 12 and the entire sliding portion 14 (cylindrical portion) including the annular projections (first projection 14a, intermediate projection 14b, and bottom-side projection 14c).

In the gasket for syringes 2 (after the formation of the surface-modified layer 21), the first projection 14a provided with the surface-modified layer 21 has a surface roughness Ra of not greater than 1.0, preferably not greater than 0.8, more preferably not greater than 0.6, in order to simultaneously achieve sliding properties and liquid leakage resistance. The lower limit of the Ra is not particularly limited, and a smaller Ra is better.

The term "surface roughness Ra" as used herein refers to the arithmetic average height Ra defined in JIS B 0601-2001 or ISO 4287-1997.

In the gasket base material 1 (before the formation of the surface-modified layer 21), the first projection 14a preferably has a surface roughness Ra of not greater than 1.0, more preferably not greater than 0.8, still more preferably not greater than 0.6, in order to simultaneously achieve sliding properties and liquid leakage resistance. The lower limit of the Ra is not particularly limited, and a smaller Ra is better.

The surface roughness Ra of the gasket base material 1 and the gasket for syringes 2 in which the surface-modified layer 21 is formed on the gasket base material 1 may be controlled, for example, by changing the surface roughness of the forming mold. Specifically, the surface roughness may be controlled by changing the particle size of the abrasive used in the final finishing step in the preparation of the mold. Examples of the abrasive include abrasive grains of diamond, alumina, silicon carbide, cubic boron nitride, boron carbide, zirconium oxide, manganese oxide, and colloidal silica. The abrasives #46 to 100 defined in JIS R 6001-1998 may be suitably used.

The material of the forming mold may be a known material, such as carbon steel or precipitation stainless steel. The forming mold may be prepared by cutting methods such as by cutting with a cemented carbide tool, coated cemented carbide, sintered cBN, or other tools, followed by polishing and finishing processes.

EXAMPLES

The present invention will be specifically described below with reference to, but not limited to, examples.

The rubber vulcanizates (gasket base materials in the form shown in Fig. 1) used in the following examples and comparative examples were prepared by crosslinking (vulcanizing at 180°C for 10 minutes) an isoprene unit-containing chlorobutyl rubber (degree of unsaturation: 1 to 2%) by triazine. In the preparation, the amounts of the filler and triazine were varied to adjust the hardness of the rubber vulcanizates so that gaskets having a Shore A hardness of 47, 50.4, 54, 57, 63, or 72 were prepared (the hardness was determined as described below).

Since it was difficult to determine the hardness of the rubber vulcanizates (gasket base materials), corresponding rubber vulcanizate sheets were prepared using the same composition and vulcanization conditions, and the hardness of the sheets was measured and used as the hardness of the gasket base materials (the hardness of the gasket base materials can be considered the same as that of the respective sheets).

[Hardness of rubber vulcanizate (gasket base material)]

The hardness (Shore A) of the rubber vulcanizates (gasket base materials) at 23°C was determined using a type-A durometer in accordance with JIS K 6253 "Rubber, vulcanized or thermoplastic - Determination of hardness".

(Example 1)

A rubber vulcanizate (gasket base material) having a Shore A hardness of 57 was immersed in a 1 wt% solution of a silane compound (Primer coat PC-3B, Fluoro Technology, the butoxy/ethoxy tetraalkoxysilane of the above formula) in butanol and taken out therefrom. The rubber vulcanizate was then left at a humidity of 90% and a temperature of 100°C for two hours to cause a reaction. The surface was washed with acetone and then water, followed by drying.

The dried rubber vulcanizate was immersed in a 2% perfluorohexane solution containing a fluoroalkyl group-containing silane compound represented by the following formula (triethoxy-1H,1H,2H,2H-tridecafluoro-n-octylsilane: T1770, Tokyo Chemical Industry Co., Ltd., fluoroalkyl group: $CF_3(CF_2)_5(CH_2)_2$-) and a perfluoroether group-containing silane compound (Daikin Industries, Ltd., OPTOOL DSX-E, a compound of formula (2)) in a ratio of 60:40 (by mass) and taken out therefrom. Thereafter, the rubber vulcanizate was left at a humidity of 90% and a temperature of 70°C for eight hours to cause a reaction. The resulting rubber vulcanizate was washed with acetone and dried. Thus, a surface-modified elastic body was prepared.

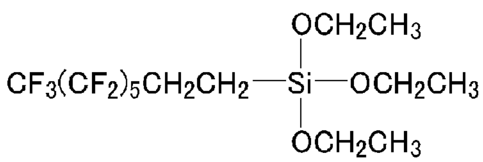

(Examples 2 to 10 and Comparative Examples 2 to 4)

A surface-modified elastic body was prepared in the same manner as in Example 1, except that the hardness of the rubber vulcanizate (gasket base material) and/or the combining ratio of the fluoroalkyl group-containing silane compound to the perfluoroether group-containing silane compound were changed as indicated in Table 1. Examples 2, 6 and 9 are not according to the invention.

(Example 11)

A surface-modified elastic body was prepared in the same manner as in Example 3, except that the fluoroalkyl group-containing silane compound was changed to $CF_3(CF_2)_3CH_2CH_2Si(OCH_2CH_3)_3$.

(Comparative Example 1)

A rubber vulcanizate (gasket base material) prepared by crosslinking (vulcanizing at 180°C for 10 minutes) an isoprene unit-containing chlorobutyl rubber (degree of unsaturation: 1 to 2%) by triazine was used as it was (Shore A hardness: 54).

The rubber vulcanizates (gasket base materials) and surface-modified elastic bodies prepared in the examples and comparative examples were evaluated as described below.

[Surface roughness Ra]

The Ra (arithmetic average height) of the rubber vulcanizates (gasket base materials) and surface-modified elastic bodies (after formation of surface-modified layer) were determined in accordance with JIS B 0601-2001 (ISO 4287-1997) .

(Thickness of surface-modified layer)

To determine the thickness of the surface-modified layer formed on the surface of the rubber vulcanizates, cross-sections of the modified rubber vulcanizates on which the surface-modified layer was formed were measured with an SEM at an accelerating voltage of 15 kV and a magnification of 1000 times.

(Sliding properties (friction resistance))

The friction resistance of the surface of the rubber vulcanizate (gasket base material) and surface-modified elastic bodies was determined as follows: the vulcanized rubber gasket prepared in each example or comparative example was inserted into a COP resin barrel of a syringe and then pushed inwardly using a tensile tester (push rate: 30 mm/min) while friction resistance was measured. The friction resistance of each example or comparative example is expressed as a friction resistance index using the equation below, with a friction resistance index of Comparative Example 1 taken as 100. A lower index indicates a lower friction resistance and better sliding properties.

$$\text{(Friction resistance index)} = \text{(Friction resistance of each example)} / \text{(Friction resistance of Comparative Example 1)} \times 100$$

(Liquid leakage resistance)

The vulcanized rubber gasket prepared in each example or comparative example was inserted into a COP resin barrel of a syringe. A solution of red food coloring in water was introduced into the barrel, and the barrel was sealed with a cap. After two-week storage at 40°C, the barrel was visually observed for liquid leakage and evaluated using the following four-point scale.

Excellent: No red (pink) stain of red food coloring was observed in the first projection nearest to the top surface.
Good: A faint red (pink) stain of red food coloring was observed in the upper half of the first projection nearest to the top surface.
Fair: A red (pink) stain of red food coloring was observed down to the bottom of the first projection nearest to the

top surface.
Poor: A red (pink) stain of red food coloring was observed beyond the first projection nearest to the top surface.

(Amount of proteins adsorbed)

The surface of the samples (rubber vulcanizate (gasket base material) and surface-modified elastic bodies) was brought into contact with a 1 mg/ml solution of bovine serum albumin (BSA) and left at 37°C for three hours. The surface of the samples was lightly washed with phosphate-buffered saline to prepare protein-adsorbed samples. The entire amount of each protein-adsorbed sample was put into a 50-ml centrifuge tube, and the proteins adsorbed on the surface of the samples were extracted in accordance with the method described in Section 3.6: Water-soluble proteins of JIS T 9010:1999 "Test methods relevant to biological safety of rubber products". To the extracted proteins was accurately added 0.5 ml of a 0.1 mol/l aqueous solution of sodium hydroxide to dissolve the proteins. Thus, sample solutions were prepared. Also, a procedural blank was prepared by the same procedure but without adding the samples.
A volume of 0.2 ml each of the sample solutions and reference solutions (5 to 100 μg/ml BSA solutions) was accurately weighed and assayed for protein amount by the Lowry method. A calibration curve was prepared from the BSA concentrations (μg/ml) and absorbances of the reference solutions. The protein concentration (μg/ml) per milliliter of the sample solution was calculated using the calibration curve, and then converted to concentration per area of the rubber vulcanizate (gasket base material) or surface-modified elastic body.
[Table 1] Examples 2, 6 and 9 are reference examples

| | Comparative Example 1 | Example 1 | Example 2 | Example 3 | Example 4 | Example 5 | Example 6 | Example 7 |
|---|---|---|---|---|---|---|---|---|
| (Shore A) hardness of gasket base material | 54 | 57 | 57 | 63 | 72 | 63 | 63 | 63 |
| Silane compound (PC-3B) | - | Present | Present | Present | Present | Present | Present | Present |
| Fluoroalkyl group-containing silane compound (T1770) /Perfluoroether group-containing silane compound (DSX-E) | - | 60/40 | 100/0 | 60/40 | 60/40 | 72/25 | 100/0 | 10/90 |
| Fluoroalkyl group-containing silane compound ($CF_3(CF_2)_3CH_2CH_2Si(OCH_2CH_3)_3$) /Perfluoroether group-containing silane compound (DSX-E) | - | - | - | - | - | - | - | - |
| Surface roughness Ra (gasket base material, before formation of surface-modified layer) | 0.45 | 0.45 | 0.45 | 0.45 | 0.45 | 0.45 | 0.45 | 0.45 |
| Surface roughness Ra (after formation of surface-modified layer) | - | 0.55 | 0.52 | 0.55 | 0.58 | 0.5 | 0.5 | 0.54 |
| Sliding properties | 100 | 3.5 | 3.7 | 2.5 | 1.5 | 3.65 | 3.8 | 3.2 |
| Liquid leakage resistance | Fair | Excellent | Excellent | Excellent | Excellent | Excellent | Excellent | Good |
| Amount of proteins adsorbed ($\mu g/cm^2$) | 1.62 | 0.34 | 0.33 | 0.33 | 0.32 | 0.33 | 0.33 | 0.45 |
| Thickness of surface-modified layer (nm) | 0 | 180 | 170 | 185 | 200 | 180 | 150 | 250 |

| | Example 8 | Example 9 | Example 10 | Comparative Example 2 | Comparative Example 3 | Comparative Example 4 | Example 11 |
|---|---|---|---|---|---|---|---|
| (Shore A) hardness of gasket base material | 72 | 72 | 72 | 47 | 50.4 | 54 | 63 |
| Silane compound (PC-3B) | Present | Present | Present | Present | Present | Present | Present |
| Fluoroalkyl group-containing silane compound (T1770) /Perfluoroether group-containing silane compound (DSX-E) | 75/25 | 100/0 | 10/90 | 60/40 | 60/40 | 60/40 | - |
| Fluoroalkyl group-containing silane compound ($CF_3(CF_2)_3CH_2CH_2Si(OCH_2CH_3)_3$) /Perfluoroether group-containing silane compound (DSX-E) | - | - | - | - | - | - | 60/40 |
| Surface roughness Ra (gasket base material, before formation of surface-modified layer) | 0.45 | 0.45 | 0.45 | 0.45 | 0.45 | 0.45 | 0.45 |
| Surface roughness Ra (after formation of surface-modified layer) | 0.48 | 0.46 | 0.52 | 0.65 | 0.62 | 0.6 | 0.554 |
| Sliding properties | 1.6 | 1.75 | 1.25 | 60 | 50 | 15 | 2.95 |
| Liquid leakage resistance | Excellent | Excellent | Good | Good | Good | Excellent | Excellent |
| Amount of proteins adsorbed ($\mu g/cm^2$) | 0.32 | 0.3 | 0.42 | 0.6 | 0.57 | 0.54 | 0.35 |
| Thickness of surface-modified layer (nm) | 185 | 160 | 285 | 150 | 155 | 160 | 150 |

Table 1 shows that the surfaces of the surface-modified elastic bodies of the examples exhibited greatly reduced friction resistance and thus good sliding properties. They also had good liquid leakage resistance and good protein adsorption resistance. Regarding the protein adsorption resistance, if the amount of proteins adsorbed exceeds 1.0 $\mu g/cm^2$, this means that the adsorption of proteins has proceeded to the multilayer state and is thus considered dominant. In the examples, the amount of proteins adsorbed was not more than 0.7 $\mu g/cm^2$, which means that the proteins are adsorbed as substantially a monolayer and their adsorption occurs simultaneously with their desorption and is thus not dominant but kept at a good level (where the adsorption does not increase any more).

Thus, when these surface-modified elastic bodies are used as gaskets for syringe plungers, they provide sufficient liquid leakage resistance while reducing the friction of the plunger against the syringe barrel, thereby enabling easy and accurate treatment with the syringes. They can also sufficiently reduce protein adsorption.

Furthermore, the above-mentioned effects can also be expected when a surface-modified layer is formed on the surfaces of the grooves formed on the tread or of the sidewalls of tires for passenger cars or other vehicles, diaphragms, the sliding surface of skis or snowboards, swimsuits, road signs, sign boards, etc.

REFERENCE SIGNS LIST

- 1: gasket base material (before surface modification)
- 2: gasket for syringes (after surface modification)
- 12: top surface
- 13: bottom surface
- 14: sliding portion (cylindrical portion)
- 14a: first projection
- 14b: intermediate projection
- 14c: bottom-side projection
- 21: surface-modified layer

## Claims

**1.** A surface modification method for modifying a surface of a rubber vulcanizate having a Shore A hardness of 55 to 90 as a modification target, the Shore A hardness being determined using a type-A durometer at 23°C in accordance with JIS K 6253 "Rubber, vulcanized or thermoplastic - Determination of hardness", the method comprising:

step 1 of adding a silane compound to the surface of the modification target; and
step 2 of reacting at least a fluoroalkyl group-containing silane compound and a perfluoroether group-containing silane compound to form a surface-modified layer.

**2.** The surface modification method according to claim 1,
wherein the fluoroalkyl group is represented by the following formula:

$$F_3C-(CF_2)_n-(CH_2)_m-$$

wherein n is 0 to 5, and m is 0 to 8.

**3.** The surface modification method according to claim 1 or 2,
wherein the fluoroalkyl group-containing silane compound is represented by the following formula (1):

$$F_3C-(CF_2)_n-(CH_2)_m-Si(OR^1)_3 \qquad (1)$$

wherein n is 0 to 5; m is 0 to 8; and each $R^1$ may be the same or different and represents an alkyl group.

**4.** The surface modification method according to any one of claims 1 to 3,
wherein the perfluoroether group-containing silane compound is represented by the following formula (2) or (3) :

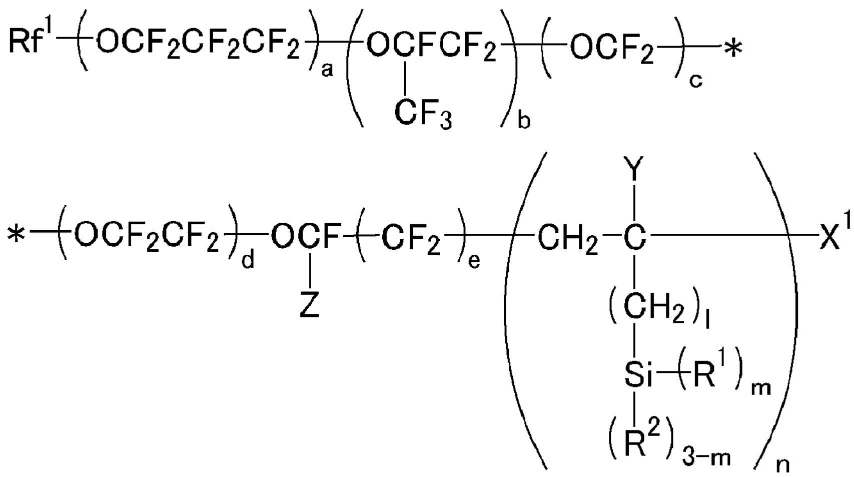

(2)

wherein $Rf^1$ is a perfluoroalkyl group; Z is fluorine or a trifluoromethyl group; a, b, c, d, and e are the same as or different from each other and each represent an integer of 0 or 1 or more, provided that a + b + c + d + e is 1 or more and the order of the repeating units parenthesized by subscripts a, b, c, d, and e occurring in the formula is not limited to that shown; Y is hydrogen or a C1-C4 alkyl group; $X^1$ is hydrogen, bromine, or iodine; $R^1$ is a hydroxy group or a hydrolyzable substituent; $R^2$ is hydrogen or a monovalent hydrocarbon group; 1 is 0, 1, or 2; m is 1, 2, or 3; n is an integer of 1 or more; and the two ends marked by * are directly bonded to each other, or

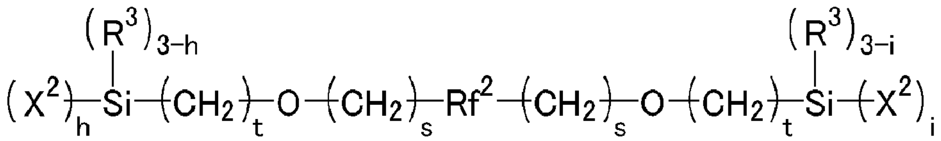

(3)

wherein $Rf^2$ is a divalent group that contains a unit represented by $-(C_kF_{2k})O-$ where k is an integer of 1 to 6, and has a non-branched linear perfluoropolyalkylene ether structure; each $R^3$ is the same or different and represents a C1-C8 monovalent hydrocarbon group; each $X^2$ is the same or different and represents a hydrolyzable group or a halogen atom; each s is the same or different and represents an integer of 0 to 2; each t is the same or different and represents an integer of 1 to 5; and h and i are the same as or different from each other and each represent 1, 2, or 3.

**5.** The surface modification method according to any one of claims 1 to 4,
wherein the surface-modified layer has a thickness of 30 to 500 nm.

**6.** A gasket for syringes, at least partly comprising the surface-modified layer formed by the surface modification method according to any one of claims 1 to 5.

**7.** The gasket for syringes according to claim 6,

wherein the gasket has a sliding surface provided with a plurality of annular projections,
the annular projections include a first projection nearest to a top surface of the gasket, and
the first projection has a surface roughness Ra of not greater than 1.0,
wherein the surface roughness Ra is the arithmetic average height Ra defined in JIS B 0601-2001 or ISO 4287-1997.

**8.** The gasket for syringes according to claim 7,
wherein the surface roughness Ra is not greater than 0.8.

**9.** The gasket for syringes according to claim 7,
wherein the surface roughness Ra is not greater than 0.6.

## Patentansprüche

**1.** Oberflächenmodifizierungsverfahren zum Modifizieren einer Oberfläche eines Kautschukvulkanisats mit einer Shore-A-Härte von 55 bis 90 als Modifizierungsgegenstand, wobei die Shore-A-Härte bestimmt ist unter Verwendung eines Typ-A-Durometers bei 23°C gemäß JIS K 6253 "Kautschuk, vulkanisiert oder thermoplastisch - Bestimmung der Härte", wobei das Verfahren umfasst:

Schritt 1 des Hinzufügens einer Silanverbindung zu der Oberfläche des Modifizierungsgegenstands; und
Schritt 2 des Umsetzens mit mindestens einer Fluoralkylgruppenhaltigen Silanverbindung und einer Perfluorethergruppen-haltigen Silanverbindung, um eine oberflächenmodifizierte Schicht zu bilden.

**2.** Oberflächenmodifizierungsverfahren nach Anspruch 1,
wobei die Fluoralkylgruppe durch die folgende Formel dargestellt ist:

$$F_3C\text{-}(CF_2)_n\text{-}(CH_2)_m\text{-}$$

wobei n 0 bis 5 ist, und m 0 bis 8 ist.

**3.** Oberflächenmodifizierungsverfahren nach Anspruch 1 oder 2,
wobei die Fluoralkylgruppen-haltige Silanverbindung durch die folgende Formel (1) dargestellt ist:

$$F_3C\text{-}(CF_2)_n\text{-}(CH_2)_m\text{-}Si(OR^1)_3 \quad (1)$$

wobei n 0 bis 5 ist; m 0 bis 8 ist; und $R^1$ jeweils gleich oder verschieden sein kann und eine Alkylgruppe darstellt.

**4.** Oberflächenmodifizierungsverfahren nach einem der Ansprüche 1 bis 3,
wobei die Perfluorethergruppen-haltige Silanverbindung durch die folgende Formel (2) oder (3) dargestellt ist:

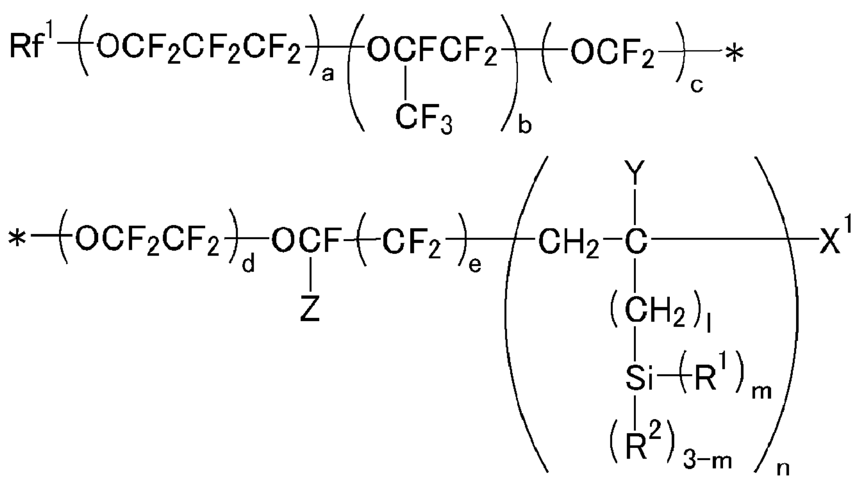

(2)

wobei $Rf^1$ eine Perfluoralkylgruppe ist; Z Fluor oder eine Trifluormethylgruppe ist; a, b, c, d und e gleich oder voneinander verschieden sind und jeweils eine ganze Zahl von 0 oder 1 oder mehr darstellen, vorausgesetzt, dass a + b + c + d + e 1 oder mehr ist und die Reihenfolge der mit Indizes a, b, c, d und e in Klammern gesetzten Wiederholungseinheiten, die in der Formel vorkommen, nicht auf diejenige beschränkt ist, die gezeigt ist; Y Wasserstoff oder eine C1-C4-Alkylgruppe ist; $X^1$ Wasserstoff, Brom oder Iod ist; $R^1$ eine Hydroxygruppe oder ein hydrolysierbarer Substituent ist; $R^2$ Wasserstoff oder eine einwertige Kohlenwasserstoffgruppe ist; l 0, 1 oder 2 ist; m 1, 2 oder 3 ist; n eine ganze Zahl von 1 oder mehr ist; und die beiden durch * gekennzeichneten Enden direkt miteinander verbunden sind, oder

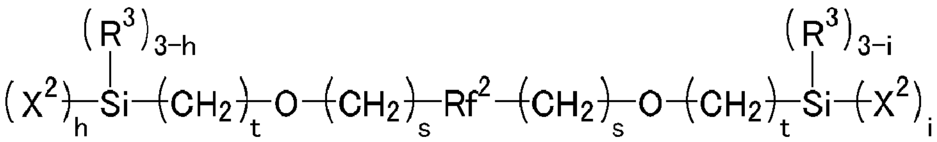

(3)

wobei $Rf^2$ eine zweiwertige Gruppe ist, die eine durch $-(C_kF_{2k})O-$ dargestellte Einheit enthält, worin k eine ganze Zahl von 1 bis 6 ist und die eine nicht-verzweigte, lineare Perfluorpolyalkylenether-Struktur aufweist; $R^3$ jeweils gleich oder verschieden ist und eine einwertige C1-C8-Kohlenwasserstoffgruppe darstellt; $X^2$ jeweils gleich oder verschieden ist und eine hydrolysierbare Gruppe oder ein Halogenatom darstellt; s jeweils gleich oder verschieden ist und eine ganze Zahl von 0 bis 2 darstellt; t jeweils gleich oder verschieden ist und eine ganze Zahl von 1 bis 5 darstellt; und h und i gleich oder voneinander verschieden sind und jeweils 1, 2 oder 3 darstellen.

**5.** Oberflächenmodifizierungsverfahren nach einem der Ansprüche 1 bis 4, wobei die oberflächenmodifizierte Schicht eine Dicke von 30 bis 500 nm aufweist.

**6.** Dichtung für Spritzen, die zumindest teilweise die oberflächenmodifizierte Schicht umfasst, die durch das Oberflächenmodifizierungsverfahren nach einem der Ansprüche 1 bis 5 gebildet ist.

**7.** Dichtung für Spritzen nach Anspruch 6,

wobei die Dichtung eine Gleitoberfläche aufweist, die mit einer Mehrzahl ringförmiger Vorsprünge versehen ist, die ringförmigen Vorsprünge einen ersten Vorsprung umfassen, der einer oberen Oberfläche der Dichtung am nächsten liegt, und
der erste Vorsprung eine Oberflächenrauigkeit Ra von nicht größer als 1,0 aufweist,
wobei die Oberflächenrauigkeit Ra die in JIS B 0601-2001 oder ISO 4287-1997 definierte arithmetische mittlere Höhen-Ra ist.

**8.** Dichtung für Spritzen nach Anspruch 7, wobei die Oberflächenrauigkeit Ra nicht größer als 0,8 ist.

**9.** Dichtung für Spritzen nach Anspruch 7, wobei die Oberflächenrauigkeit Ra nicht größer als 0,6 ist.

## Revendications

**1.** Procédé de modification de surface pour modifier une surface d'un vulcanisat de caoutchouc ayant une dureté Shore A de 55 à 90 comme cible de modification, la dureté Shore A étant déterminée en utilisant un duromètre de type A à 23 °C selon la norme JIS K 6253 « Caoutchouc, vulcanisé ou thermoplastique - détermination de la dureté », le procédé comprenant :

l'étape 1 d'addition d'un composé silane à la surface de la cible de modification ; et
l'étape 2 de mise à réagir d'au moins un composé silane contenant un groupe fluoroalkyle et d'un composé silane contenant un groupe perfluoroéther pour former une couche modifiée en surface.

**2.** Procédé de modification de surface selon la revendication 1, dans lequel le groupe fluoroalkyle est représenté par la formule suivante :

$$F_3C\text{-}(CF_2)_n\text{-}(CH_2)_m\text{-}$$

dans laquelle n vaut de 0 à 5 et m vaut de 0 à 8.

**3.** Procédé de modification de surface selon la revendication 1 ou 2, dans lequel le composé silane contenant le groupe fluoroalkyle est représenté par la formule (1) suivante :

$$F_3C\text{-}(CF_2)_n\text{-}(CH_2)_m\text{-}Si(OR^1)_3 \qquad (1)$$

dans laquelle n vaut de 0 à 5 ; m vaut de 0 à 8 ; et chaque $R^1$ peut être identique ou différent et représente un groupe alkyle.

**4.** Procédé de modification de surface selon l'une quelconque des revendications 1 à 3, dans lequel le composé silane contenant le groupe perfluoroéther est représenté par la formule (2) ou (3) suivante :

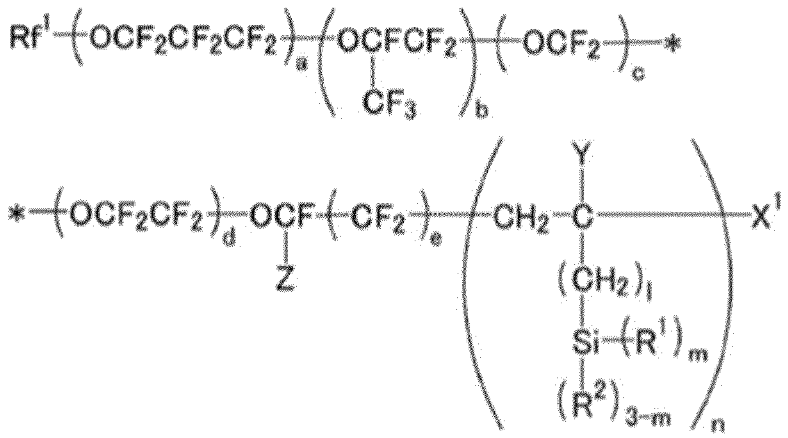

(2)

dans laquellle $Rf^1$ est un groupe perfuoroalkyle ; Z est un atome de fluor ou un groupe trifluorométhyle ; a, b, c, d et e sont identiques ou différents l'un de l'autre et chacun représente un nombre entier de 0 ou 1 ou plus, pourvu que a+b+c+d+e vale 1 ou plus et l'ordre des motifs répétés entre parenthèses par les indices a, b, c, d et e se produisant dans la formule n'est pas limité à celui montré ; Y est un atome d'hydrogène ou un groupe alkyle en C1 à C4 ; $X^1$ est un atome d'hydrogène, de brome ou d'iode ; $R^1$ est un groupe hydroxyle ou un substituant hydrolysable ; $R^2$ est un atome d'hydrogène ou un groupe hydrocarbure monovalent ; l vaut 0, 1 ou 2 ; m vaut 1, 2 ou 3 ; n est un nombre entier de 1 ou plus ; et les deux extrémités marquées par * sont directement liées l'une à l'autre, ou

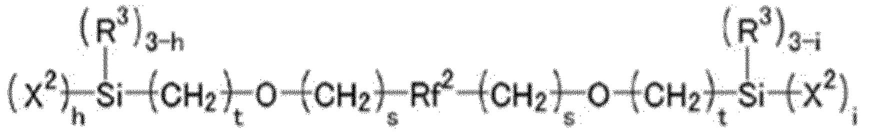

(3)

dans laquelle $Rf^2$ est un groupe divalent qui contient un motif représenté par $-(C_kF_{2k})O-$ où k est un nombre entier de 1 à 6 et a une structure d'éther de perfluoropolyalkylène linéaire non ramifiée ; chaque $R^3$ est identique ou différent et représente un groupe hydrocarbure monovalent en C1 à C8 ; chaque $X^2$ est identique ou différent et représente un groupe hydrolysable ou un atome d'halogène ; chaque s est identique ou différent et représente un nombre entier de 0 à 2 ; chaque t est identique ou différent et représente un nombre entier de 1 à 5 ; et h et i sont identiques ou différents l'un de l'autre et chacun représente 1, 2 ou 3.

**5.** Procédé de modification de surface selon l'une quelconque des revendications 1 à 4,
dans lequel la couche modifiée en surface a une épaisseur de 30 à 500 nm.

**6.** Joint d'étanchéité pour seringues, comprenant au moins partiellement la couche modifiée en surface formée par le procédé de modification de surface selon l'une quelconque des revendications 1 à 5.

**7.** Joint d'étanchéité pour seringues selon la revendication 6,

dans lequel le joint d'étanchéité a une surface de glissement munie avec une pluralité de projections annulaires, les projections annulaires comprennent une première projection la plus proche d'une surface de partie supérieure du joint d'étanchéité, et
la première projection a une rugosité de surface Ra de pas plus de 1,0,
la rugosité arithmétique Ra étant la hauteur moyenne arithmétique Ra définie dans la norme JIS B 0601-2001 ou ISO 4287-1997.

**8.** Joint d’étanchéité pour seringues selon la revendication 7, dans lequel la rugosité de surface Ra est de pas plus de 0,8.

**9.** Joint d’étanchéité pour seringues selon la revendication 7, dans lequel la rugosité de surface Ra est de pas plus de 0,6.

FIG. 1

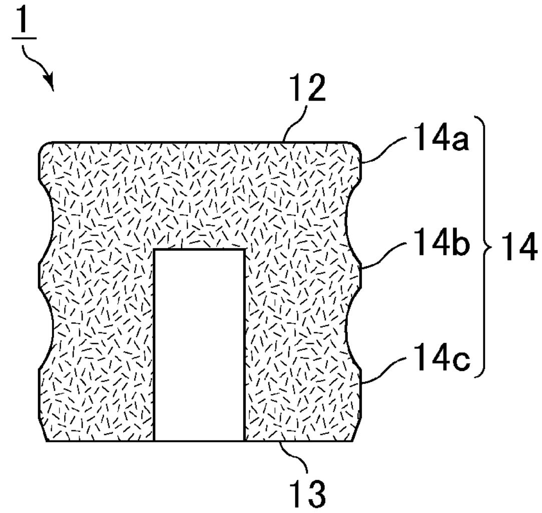


FIG. 2

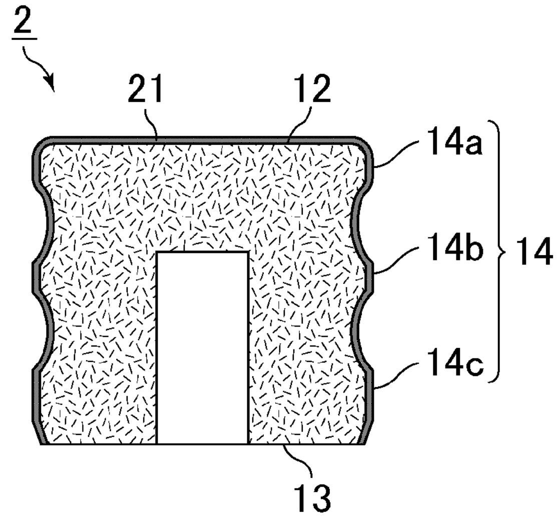

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3173106 A
- JP 2004298220 A
- JP 2010142573 A
- EP 3173106 A1